# EUROPEAN PATENT APPLICATION

(11) **EP 1 279 388 A1**
(43) Date of publication of application: **29.01.2003**
(21) Application number: 01117669.0
(22) Date of filing: 26.07.2001
(51) Int. Cl.: A61F 13/15

(54) **Absorbent article with cuffs**

(71) Applicant: THE PROCTER & GAMBLE COMPANY, Cincinnati, Ohio 45202 (US)
(72) Inventor: Müller, Jörg, 61184 Karben (DE); Westerheide, Lars, 65779 Kelkheim (DE); Schmidt, Mattias, 65510 Idstein (DE)
(74) Representative: Hirsch, Uwe Thomas M.H.

(57) **Abstract**

The invention relates to absorbent articles, such as diapers, training pants, adult incontinence articles, feminine protection articles and the like having a specific anal or vaginal cuff with an opening, which is sag-tolerable. The articles have a specific construction of a core, backsheet and the cuff, which each have specific dimension, in relation to one another. Preferred is for example that that the cuff has elasticated regions along the opening and that the cuff is (non-elastically) extendible is transverse direction, having for example longitudinal folds which easily unfold and extend the cuff in transverse direction, ensuring the cuff does not sag when the backsheet and care sack due to soiling.

## Description

### FIELD OF THE INVENTION

This invention is directed to absorbent articles, such as diapers, training pants, adult incontinence articles, feminine protection articles and the like having a specific anal of vaginal cuff.

### BACKGROUND OF THE INVENTION

Wearable absorbent articles, such as diapers, are well known in the art. These articles typically have an absorbent core, which is held or positioned against the body of the wearer during use by a fastening system, such that the bodily exudates are caught by the article. Typical absorbent articles include a cuff facing the wearer, which permits fluid exudates to pass through, and a backsheet, which prevents the exudates from escaping from the absorbent article.

Much advancement has been made in the art since the introduction of the disposable absorbent article. However, problems still exist relating to the acceptance and storage of bodily fluids, e.g. such that they do not stay completely on top of the article and in contact which the wearer's skin, e.g. such that the majority of the bodily fluid is removed from the cuff, away from the skin. The problem is particularly difficult to solve if large amounts of fluid are present and/ or because feces generally will not pass through a cuff and thus, remains free to move about in the diaper until the diaper is changed. This often leads to feces escaping the diaper or soiling of the wearer's skin. In particular fluid feces has this problem, since it is very mobile on the cuff and easily moves from one side to another and easy escapes the diaper's leg portions or leg cuffs.

In order to prevent the feces from escaping the absorbent article or soiling the skin, apertures have been provided in the top sheet, which allow the feces to pass to a void space disposed between the cuff and underlying layers of the diaper. However, the apertures are difficult to position during application of the article and often move from the desired position when the article is worn.

Some degree of success has been achieved using an elastically foreshortened cuff having a generally elliptical aperture to allow feces passage and retention away from the skin. These approaches have the limitation of not maintaining alignment of the opening with the wearer's anus in one or more of the longitudinal, lateral or z-direction axes of the article.

Minor improvements have been suggested in the prior art by providing differently shaped apertures and elastic bands along the apertures. These solutions may improve the initial alignment of the aperture and the anus and may ensure the cuff with the aperture is in contact with the wearer's anus, when the diaper is put on. However, the diapers with apertures proposed in the prior art tend not to have satisfactory performance is practice, and are not commercialized.

The inventors have now found that the poor performance of the prior art proposed apertures diapers is to a very large extend caused by the increasing, built-up weight of the bodily fluids stored in the article during use, which results in a movement of the article, e.g. down wards, away from the skin of the wearer.

The inventors have now found an alternative way to provide absorbent articles which can receive and store bodily fluids and keep these away from the skin, even during the whole usage of the article, namely by providing an article with has an specific anal cuff which is sag-tolerable, e.g. which has a means to remain in contact with the wearer's skin when the backsheet and/ or core of the diaper sag due to increased gravity forces on the increasing weight of the exudates. The inventors found that a preferred article thereto has a specific backsheet, core and anal or vaginal cuff construction, with specific dimensions in relation to one another. The cuff can remain in position and does not sag when the weight of the collected bodily fluids in the article pulls the backsheet down, due to the specific dimensions of the cuff, the backsheet and the specific geometrical relationship there between.

The inventors also found that the problem can be solved by providing an anal or vaginal cuff, which has a means to stay in close proximity to the wearer's skin and which has a specific extendibility, e.g. which is extendible by application of a very small force, typically non-elastically, whereby the whole cuff is extendible in transverse direction when the remaining part of the article start to sag. The inventors found that it is highly preferred that the cuff has thereto longitudinal folds, which can unfold when a force is applied to the cuff, such as the pulling force of the backsheet or core.

### SUMMARY OF THE INVENTION

The invention provides in a first embodiment an absorbent article having a backsheet, a core, and a sag-tolerable anal cuff and/ or vaginal cuff, which contains an opening.

The article of the invention is preferably such that it has a shortened article portion, which has a shortened article length L and a stretched shortened article length Lₛ, and whereby the backsheet, cuff and core each have a transverse axis and a longitudinal axis, whereby the height H₁ from the geometrical center point of the core, which is in a fixed position, fixed along its transverse axis, to the geometrical center point of the cuff, pulled away from the core in the direction of the height H₁ with a force of 1.0N or even 0.2N, is at least 0.25Lₛ, preferably at least 0.35Lₛ.

Alternatively, the article of the invention is such that it has a shortened article portion, which has a shortened article length L and a stretched shortened article length Lₛ and whereby the backsheet, cuff and core each have a geometrical center point (the cross point of each transverse axis and a longitudinal axis) whereby the height H₂ from the geometrical center point of the core, which is in a fixed in position, fixed by its geometrical center point, to the geometrical center point of the cuff, pulled away from the core in the direction of the height H₂ with a force of 1.0N or even 0.2N, is at least 0.3Lₛ, preferably at least 0.4Lₛ.

In yet another alternative embodiment, the article of the invention is such that it has a shortened article portion, which has a shortened article length L, a stretched shortened article length Lₛ and whereby the backsheet, core and cuff each have a longitudinal axis and a transverse axis and whereby each longitudinal edge of the cuff, or part thereof and a corresponding longitudinal edge of the backsheet is connected to one another to form a longitudinal connection area and whereby the backsheet has a geometrical center point A, and the cuff has a geometrical center point D and whereby each connection area has an inner connection line, being the line closest to said center point A, and an outer connection line, being the closest to the point D, whereby the transverse axis through A intersects the inner connection line in a point B on the backsheet and the transverse axis through D intersects the outer connection line in point C, whereby the distance H₃, being the distance (A to B) + (C to D) is at least 0.3Lₛ, preferably at least 0.5Lₛ.

Preferred is that the article is in the form of an infant or adult diaper or infant training pant or pull-on pants, which has a contracted shortened article portion with a length L_{c} whereby in use L_{c} is smaller than the shortest distance Lᵣ between the belly button and the small of the back of the infant or adult (also referred to as the 'rise' of a person), preferably L_{c} being at least 20% less than Lᵣ. The performance of this sag-tolerable diaper or pants can for example be tested with a baby or a mannequin baby or doll and artificial exudates.

The cuff, in relaxed state, is preferably longitudinally extendable and also transversely extendible when a force 1N or less is applied to the cuff, e.g. non-elastically extendible. Preferably, the cuff has thereto, in relaxed state, one or more longitudinal folds.

The slit opening of the cuff preferably contains one or more elasticated regions and/ or adhesive regions along each longitudinal edge of the opening, whereby preferably the elasticated regions each have one or more elastic bands longitudinally along said region, having each a width of 5 to 30 mm, preferably 10 to 20mm.

Furthermore, the inventors found that it is advantageous not to attach the longitudinal edges of the crotch region of the anal cuff and the core, and thus decoupling the cuff from the core, which stores the bodily fluids and becomes heavy during use. Hereby, the pulling force from the bodily fluids only pulls down the core and part of the backsheet, but not the cuff, in particular when this is extendible. Thus, the cuff can remain in close proximity with the skin and the bodily fluids continue to be effectively collected by the article, e.g. through the slit into the void space.

Thus, preferably, the core is positioned between the cuff and backsheet and the core and the cuff are not joined to one another.

The cuff may be a vaginal cuff of a diaper or feminine protection articles, such as sanitary napkins, or more preferably the cuff is an anal cuff and the article is an adult or infant diaper, or pull-on pants or training pants.

In the diapers of the invention, the anal cuff may equally serve to receive urine, so when worn by females, the cuff may in effect be an anal and vaginal cuff at the same time.

### BRIEF DESCRIPTION OF THE FIGURES

Figure 1 is a plan view of a disposable diaper configuration of the present invention.
Figure 2 is perspective view of the diaper configuration shown in Figure 1.
Figure 3 is a perspective view of a diaper as in figure 1 and 2, when a force is applied onto it.
Figure 4a and 4b are transverse cross-sections of the diaper of figures 1, 2 and 3 in use, i.e. when initially applied and after being soiled in use.

### DETAILED DESCRIPTION OF THE INVENTION

As used herein, the following terms have the following meanings:

"Absorbent article" refers to wearable devices, which absorb and/ or contain liquid, and more specifically, refers to devices, which are placed against or in proximity to the body of the wearer to absorb and contain the various exudates discharged from the body.

As used herein, the term "diaper" refers to an absorbent article generally worn by infants and incontinent persons about the lower torso.

When used herein, longitudinal" is generally a direction running parallel to the maximum linear dimension, typically the longitudinal axis, of the article and includes directions within 45° of the longitudinal direction. 'Length' of the article or component thereof, when used herein, generally refers to the size/ distance of the maximum linear dimension, or typically to the size/ distance of the longitudinal axis, of an article or part thereof.

The "lateral" or "transverse" direction is generally orthogonal to the longitudinal direction, e.g. in the same plan of the majority of the article and the longitudinal axis, and the transverse direction is parallel to the transverse axis. 'Width' of the article or of a component thereof, when used herein, refers to the size/ distance of the dimension orthogonal to the longitudinal direction of the article or component thereof, e.g. orthogonal to the length of the article or component thereof, and typically it refers to the distance/ size of the dimension parallel of the transverse axis of the article or component.

'Thickness' of the article or component thereof, when used herein, refers to the size/distance of the dimension orthogonal to both the longitudinal and transverse directions, e.g. running parallel to the minimum linear dimension of the article, i.e. the "Z-direction" dimension.

When used herein, the 'geometrical middle point' of the article or component thereof, such as the cuff, backsheet or core, is the cross point of the longitudinal and transverse axis of the article or component.

'Extendibility' and 'extendible', e.g. extendibility of the cuff, when used herein means that the width or length of the item in relaxed position can be extended or increased.

When used herein, 'larger extendibility' referring to the extendibility of one item compared to another, means that the absolute extendibility of the first item is more than the absolute extendibility of the other item.

As used herein, the term 'attached' encompasses configurations whereby an element is directly secured to another element by affixing the element directly to the other element.

As used herein, the term 'joined' or 'connected' encompasses configurations whereby an element is directly secured to another element by affixing the element directly to the other element and configurations whereby an element is indirectly secured to another element by affixing the element to intermediate member(s), which in turn are affixed to the other element.

As used herein, the term "void space" is a cavity sized in the article present in at least the relaxed state, which serves to accept and contain bodily exudates such as fecal material.

As used herein 'relaxed' or 'relaxed state' means the state that no forces are applied to the article (other than naturally occurring forces such as gravity), when the article is laid on a horizontal surface; this includes the state of the article whereby L equals L_{c} (as defined below).

As used herein, 'stretched' or stretched state' means that the article is stretched to the point that the shortened article portion has the length Lₛ (as defined below).

As used herein, 'elasticated' means, that the component (e.g. anal or vaginal cuff) comprises at least a portion made of elastic material, which is elastic in at least the longitudinal direction, including the situation that the component e.g. cuff, as a whole is of elastic materials (elastically extendible in longitudinal direction) and/ or that the component, e.g. cuff, contains elastic bands or strands, elastically extendible in longitudinal direction.

As used herein, 'along the longitudinal axis' means at least partially parallel to the longitudinal axis.

The absorbent article of the invention has an anal or vaginal cuff, which is sag-tolerable. This means that the cuff does not substantially sag and that the cuff keeps its z-direction alignment with the anal region or vaginal region of the wearer when the remaining components of the article, namely the backsheet and core, sag due to increased weight of body exudates received by the article. It thus has means to stay in about the same contact or close proximity with the wearer's anal or vaginal region when the backsheet and/ or care sag, compared to when the article is just put on the wearer, and the backsheet and core do not yet sag. Typically the cuff is sag-tolerable to such an extend that when the geometrical point of the backsheet is pulled down with 4cm, the anal cuff does not move down with more than 0.5cm, or even not more than 0,25cm, or typically the cuff does not move down at all. Typically the absorbent article (preferably a diaper or training pants or pull-on pants) is sag-tolerable, fulfilling the requirements as defined in any of the dependent claims herein.

The cuff of the article herein has an opening, preferably an elongated split opening. This opening typically leads to a void space, present at least when the article is in relaxed state, which can contain the bodily fluids, e.g. feces. Preferred openings are described herein after.

The article's backsheet, core and cuff have each a longitudinal direction and a transverse direction and a z-direction, a longitudinal axis and a transverse axis and a geometrical center point.

The cuff, core and backsheet each have a first waist region, a second waist region and a crotch region, positioned therein between, such that they have a mutual longitudinal axis (these 3 regions being transverse positioned, so that they together form part or all of the length of the article).

The absorbent article of the invention has a shortened article portion with a shortened article length L, a stretched shortened article length Lₛ and a contracted shortened article length L_{c}.

The shortened article portion is the portion of the article where the existence of the specific force profiles as defined herein are in particular beneficial, typically the portion of the article between the force lines in use, e.g. if the article is a diaper, worn by a (fastened) waistband around the waist of the wearer, who is in upright position, then the front force line is typically the horizontal line were the front waist band starts (i.e. the lowest point) and the back force line is the horizontal line were the back waist band starts (i.e. the lowest point), and the shortened article portion is the portion of the diaper between these to force lines. Because it is often difficult to determine the exact location of the force lines, the shortened article portion is for the purpose of the invention defined as follows.

The shortened article portion is determined by removing from each transverse end of the article a transverse strip with a width (e.g. the dimension parallel to the longitudinal axis of the article) of 20% of the articles total length (in relaxed state), so that the shortened article portion is the middle 60% of the article (in relaxed state). The shortened article length is then the length of the longitudinal axis of the shortened article, e.g. about 60% of the article length.

Even more preferred may be that the shortened article portion is the middle 40% of the article, thus, the article whereof on each transverse end a transverse strip of 30% of the length of the article is removed, and the shortened article length for an article herein is then typically 40% of the length of the article.

The stretched shortened article length Lₛ is determined as follows:

The article is placed between to clamps in a horizontal tensile tester Z10/LH 1S, as available from Zwick (Ulm, Germany). The clamps have at least the same size as the width of the article, so that the clamps at least cover the total width of the article.

The clamps are positioned such that exactly the shortened product portion is between the clamps and such that exactly (and only) the shortened product length is not covered by the clamps. The initial clamp distance should then be 4cm. The measurement is done in a controlled environment, whereby the temperature is kept constant on 20°C and the humidity on 30%. The article is then pulled in horizontal, longitudinal direction up to the moment that a force of 20N is applied. Then, the distance between the clamps and thus between the transverse ends of the shortened article portion is measured. This is the stretched shortened article length Lₛ.

The contracted shortened article length L_{c} is determined as follows.

After the measurement of Lₛ above is done, the article is rested for an hour, in the controlled conditions set out above. Then, whilst still under the controlled conditions, the article is placed in the top clamp of a vertical tensile tester (as available from Zwick). On the other end a clamp with a weight of 10 grams is placed, but still supported so that the weight does not start pulling yet due to gravity.

The clamps are positioned such that exactly the shortened product length is not covered by the clamps and thus that the end of the clamps are positioned exactly at the ends of the shortened article portion. The clamps have at least the size of the width of the article at the clamping point, so that the clamps cover the total width of the article.

Then, the support for the weight is removed and the weight is hung down for 5 minutes. Then, the distance between the clamps and thus the distance between the ends of the shortened article portion is measured. This is the contracted shortened article length L_{c}.

In one embodiment, the article has a specific height H₁, which is at least 0.25 Lₛ, more preferably 0.3 Lₛ, or even 0.35 Lₛ or even 0.4 Lₛ or even 0.45 Lₛ. H₁ can be determined as follows.

The article's core has two longitudinal edges and a transverse axis, which intersects with the two longitudinal edges in two intersections. The core is fixed into a fixed, horizontal position by attaching the two intersection points to a horizontal flat surface, namely by two intersection areas each of about 0.5cm extending in both longitudinal directions from the actual intersection point. Thus, the core is fixed in transverse direction and can thus not fold around a longitudinal axis, for example.

The cuff then lies on top of the core, e.g. not facing the surface.

Then, the cuff is pulled upwards, along a force line perpendicular to the core, e.g. vertically upwards, along the height H₁, with a force of 1 N or less, preferably even 0.2N or less. Hereby, the cuff is pulled upwards by pulling the geometrical center point upwards; if the geometrical center point is located in the opening of the cuff, then the cuff is pulled upwards by the two point located on the cuff, which are in transverse direction closest to the geometrical center point of the cuff. The pulling can be done by any means, for example by attaching a small hook to the relevant point (s) of the cuff and puling the hook upwards, while measuring the force applied, as to not exceed the above specified forces.

The H₁ is than the shortest distance between the geometrical center point of the core and the pulled up geometrical center point of the cuff.

The above-mentioned forces are chosen such that the cuff is only straightened or extended, without elastically deforming and elastically extending.

This measurement method to determine H₂ is in particular applicable when the article has longitudinal stiff core.

In another embodiment of the invention, the article has a specific height H₂, which is at least 0.3 Lₛ, more preferably 0.35 Lₛ, or even 0.4 Lₛ or even 0.45 Lₛ or even 0.5 Lₛ. H₂ can be determined as follows.

The core's geometrical center point is determined and an area of 1cm² is marked around this center point. The core is fixed into a fixed, horizontal position by attaching this 1cm² to a horizontal flat surface. Thus, the core is centrally fixed. The cuff is on top of the core, e.g. not facing the surface.

The fixed core is then folded along its longitudinal axis, so that the core is generally folded upon it self (and the two halves are in contact with one another), and pressed with a force of 100N.

Then, the cuff is pulled upwards, along a force line perpendicular to the core, e.g. vertically upwards, along the height H₁, with a force of 1 N or less, preferably even 0.2N or less. The application of this force and the puling upwards of the cuff is done as above.

The H₂ is than the shortest distance between the geometrical center point of the core and the pulled-up geometrical center point of the cuff.

The above- mentioned forces are chosen such that the cuff is only straightened or extended, without elastically deforming and elastically extending.

This measurement method to determine H₂ is in particular applicable when the article has a core, which is longitudinally bendable, as is the case with the cores used in most diapers, when the legs of the user put pressure on the core and thereby bend the core. Then, this method resembles thus at best the real in-use situation.

In another preferred embodiment of the invention, the article has a specific height H₃, which is at least 0.3 Lₛ, more preferably 0.35 Lₛ, or even 0.4 Lₛ or even 0.45 Lₛ or even 0.5 Lₛ.
H₃ can be determined as follows.

The article submitted to this measurement has a backsheet and cuff which are connected to one another along their longitudinal edges, i.e. at each side, at least the portion of the longitudinal edge of the backsheet at the intersection of the transverse axis of the backsheet and said edge is attached to at least the portion of the longitudinal edge of the cuff at the intersection of the transverse axis of the cuff and said edge, and typically the whole length of the edges are attached. Thus, two connection areas are formed which each have a inner connection line, which is the longitudinal edge line of the connecting area, closest to the geometrical center point A of the backsheet (which is also determined for this measurement, by the manner defined above), and an outer connection line, which is the opposite longitudinal edge line of the connecting area.

The transverse axis through point A intersects each connection edge in point B. Each point B has a corresponding point C on the cuff, that are the intersection points of the transverse axis of the cuff and the outer connecting line. Also the geometrical center point D of the cuff is determined. H₃ is then distance (A to B) plus the distance (C to D).

The connection referred to above may be a very thin connection area, formed when the cuff and backsheet are placed next to one another and then connected, and then the inner connetion line and outer connection line are about identical and point B and C are typically about the same point; or the connection area may be wider, formed by forming an area of overlap between the cuff and the backsheet and then connecting the overlapping area. It may also be the case that the cuff and backsheet are not directly connected to one another, but that one or more other components of the article indirectly connect(s) the cuff and the backsheet. Then, the connecting area is typically the whole area between the longitudinal line or area where the backsheet is connected to this other component, up to the line where the cuff is connected to this component, and the inner and outer connecting lines of og connecting area are then as defined above, i.e. the closest line to the geometrical center point of the backsheet A and the closest line to the geometrical center point of the cuff D, respectively.

In each of the above methods, the measurements are done on a substantially flat material, which is however not elastically extended.

For example, in a preferred embodiment herein the cuff has longitudinal folds, which unfold non-elastically, and in that case, the distance from C to D is the absolute distance, i.e. the distance of the flattened out cuff.

In a preferred embodiment, the article of the invention is an adult or infant diaper with a 'rise' Lᵣ, which is the shortest distance Lᵣ from the belly button of the wearer to the small of the back of the wearer, measured via the crotch, which is in use larger than L_{c}, preferably such that L_{c} is at least 20% less than Lᵣ, more preferably at least 30% or even at least 40% or even at least 50% or even at least 60%.

'In use' means that this is the case when the diaper is first put on, as well as during use and when the diaper comprises bodily fluids, in particular fecal matter.

The above measurements of Lᵣ may be done on a baby or a baby mannequin (doll), for example, a mannequin resembling a baby suitable to wear size 4 diapers, having a thigh circumference of 28 cm, a crotch crease over the hip of 35 cm and a waist circumference of 46cm.

In a preferred embodiment of the invention, the article has a L_{c} which is less than 0.5Lₛ of the article. Preferably, L_{c} of the article is less than 0.45Lₛ of the article, or even less than 0.4Lₛ, or even less than 0.35Lₛ, or even less than 0.3Lₛ.

The article of the invention is preferably such that the maximum length of the opening in the cuff is from 50% of L_{c}, to 90% of L_{c}, or even from 60% or even 70% to 80% of L_{c}, Lc being as defined above.

The maximum length of the cuff's opening equals the length of the opening when the opening is rectangular, or equals the largest diameter when the opening is oval; or when the opening is hexagonal shaped, as is a preferred embodiment herein, the maximum length is measured from the top of one triangular portion, forming one end of the opening, to the top of the opposite triangle, forming the other end of the opening.

Preferred may also be that the article of the invention is such that the ratio of the longest width of the cuff in transverse direction to Lₛ is at least 1:5, preferably at least 1:4 or even at least 1:3.

In a preferred embodiment of the invention, the cuff has in longitudinal direction 3 parts, a first 1/3 part which is the first waist region, a second or middle 1/3 part which is the crotch region and a last 1/3 part which is the second waist region. The 3 parts may not be equal in length or width, but in one embodiment, the 3 parts are about equal in longitudinal dimension, each being about 1/3 of the total length of the article.

In one embodiment of the invention, the cuff is transversely extendible in all three parts, such that the extendibility is larger in the middle part than in the first and last parts. Preferred may be that the middle part has an absolute extendibility which is 1.5 times or even 2.5 times as much as the first and last part. Preferably, the middle part has a larger relative transverse extendibility, compared to the first and last parts. Preferred may be that the relative transverse extendibility of the cuff in the middle part is 1.5 times or even 2.0 times more than the relative extendibility in the first and last part.

In another embodiment of the invention, the cuff is transversely non-elastically extendible, and preferably in such a manner as described above. This means that when the cuff is extended in transverse direction, the elastic forces counter acting this extension are pulling the cuff back into the original position, are neglectable, e.g. when the cuff is transversely extended with a certain force and this force is released, the extension of the cuff remains for at least 80% or even at least 90%. Typically the non-elastically extendibility is such that when a 1N or even 0.2N force is applied on the geometrical center point of the cuff, the cuff is extended to its maximum extendibility.

The cuff, in relaxed position, has a (relative) width, which is smaller in that relaxed position than when a force is applied on the cuff or the article, e.g. the width of the cuff and thereby preferably the surface area of the cuff increases when the article is no longer in the relaxed position. In one embodiment of the invention, and preferred in other embodiments of the invention, this is done in a non-elastic manner.

Preferably, the cuff may have in relaxed position a relative width (in each of the 1/3 parts herein) and in extended position an absolute width (the width when a force is applied on the cuff or article and the cuff or article is thus no longer in relaxed position), whereby the relative width is smaller than the absolute width. Thereto, the cuff preferably has one or more folds in longitudinal direction, as described hereinafter. Preferred may be that the cuff has more folds or larger folds in the middle part or crotch region than in the other two part (both waist regions), to provide a relative larger extension in the middle part/ crotch region, compared to the first and second parts/ waist regions.

In another preferred embodiment of the invention, an absorbent article is provided which has a cuff and backsheet with respectively a width Wc and Wb, such that Wc is larger than Wb. Wc is the (absolute) width or extended with of the cuff in the geometric center point of the cuff, and Wb is the absolute width of the backsheet in its geometric middle point of the backsheet. The Wc is typically at least 10% more than Wb, preferably at least 25%, or even at least 35% or even at least 40% or even more than50% more than Wb.

Also preferred may be that the article is such that, when the backsheet is attached along its longitudinal axis to a horizontal flat, planar surface and the geometrical center point of the cuff is pulled vertically upwards, with a force along the substantially vertical axis though the geometrical center point of the cuff and backsheet, the force being such that no elastic deformation of the backsheet or cuff occurs, that then the distance H_{c} from the geometrical center point of the cuff to the geometrical center of the backsheet is more than Wb-4cm, and/ or that then H_{c} is 0.8Wb or more, preferably 1.0Wb or more, 1.2Wb or more, or even 1.4Wb or more.

In a preferred embodiment of the invention, the article herein has an opening, preferably an elongated split opening, preferably in at least relaxed state leading to a void space, whereby the opening has longitudinal edges, which each have one or more elasticated regions. The elasticated regions further help to ensure that the cuff remains in close proximity or contact with the wearer during use.

The elastic regions may be in the form of a single strand or band of elastic material, or multiple strands or bands.

Preferred elastic materials used hereto include materials having an elastic profile like VFE-CD, available from Tredegar, and L-86, available from Fulflex (Limerick, Ireland), or preferably L-89, available from Fulflex, or most preferred are of course one or more of these materials itself.

The materials typically have a thickness (e.g. gauge) of at least 20 microns, more preferably at least 40 microns, or even at least 70 microns, typically up to about 300 microns, or even up to 200 microns or even up to 150 microns. Highly preferred materials have a thickness of about 70 to 100 microns.

The length and width of the elastic regions along the opening of the cuff will vary, typically depending on the exact dimensions of the cuff and/ or the article.

Preferably, the elasticated regions each have one or more elastic bands longitudinally along said region, having each a width of 5 to 30 mm, preferably 10 to 20mm.

For example, for size 4 diapers, the elastic region, in relaxed state, may preferably be 8 to 30mm, or even 10 or even 8 to 25mm.

Preferred is that the opening is positioned in at least the crotch region of the cuff, which is to be aligned with the anus or vagina; preferably the opening of an anal cuff is configured such that from 0%, or even from 10%, or even from 20% to 40% or even to 30% of the length of the opening extends from the transverse axis of the cuff or article towards the first or front waist region, to be positioned at the front of the wearer.

The dimensions of the opening may vary, depending on the size of the cuff and/ or the article. Preferred may be, in particular for size 4 diapers, that the length of the opening, e.g. in relaxed state is preferably from 5 to 30cm, or even from 10 to 25 cm, or even from 12 to 20cm. The width of the opening of such articles, e.g. in relaxed state, is preferably from 2 to 10cm, more preferably 3 to 8cm.

In a stretched state, the length of the opening may preferably be from 20 to 35 cm, and the width may preferably be from 3 to 6 cm.

The elasticated regions are preferably positioned along the two longitudinal edges of the opening (so that each edge has at least one elasticated region), extending from said opening towards the first (front) and second (back) waist region, preferably such that the end portions of the elastic regions can be attached or joined to the waist region. Thus, the elasticated regions are preferably longer than the opening, both in relaxed as in stretched state. Preferred is that the elastic region is positioned over the about full length of the cuff, minus the parts of the cuff which form (part of) the waist region or band.

The length of the elastic region will typically depend on the size of the article. For example, for a size 4 diaper, the length of the elastic region in relaxed position may be 10 to 30 cm, or even 15 to 25, whilst in stretched position preferably from 20 to 60 or even 25 to 45 or even 30 to 40cm.

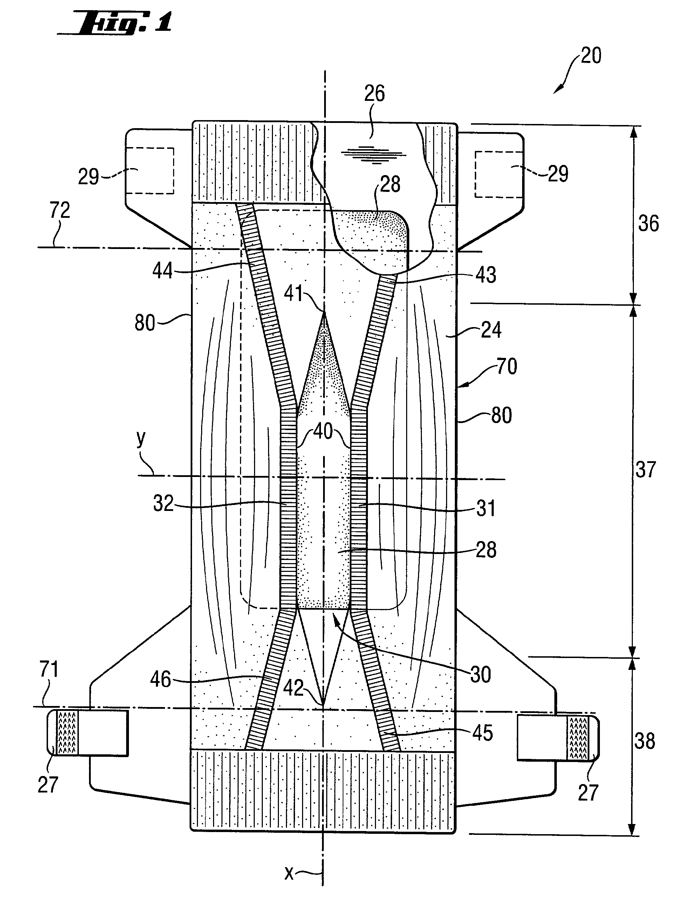

The elastic region may be in the form of two or more substantially parallel elasticated zones, and preferred is that the elasticated regions are shaped such that the middle portions of the regions are substantially parallel to one another, whist the end portions (at least in relaxed state) bend away from one another (in the plane of the cuff), so that the distance between the end portions of the elastic regions is larger that the distance between the middle portions of the elastic regions. Then, the end portions of the elasticated regions make typically each an angle with the longitudinal axis of the opening, preferably each angle being between 20° and 30°, and preferably such that the angle between the end portions is about twice as much, e.g. between 40° and 60°. This is herein referred to as an X-shape, and a preferred X-shape is exemplified in Figure 1, as described herein after. In this preferred embodiment in contracted state, the preferred maximum distance between the elastic regions along each longitudinal edge of the opening is at least 150% of the minimum distance between the elastic regions.

Preferred is that, when in relaxed state, the elastic regions or part thereof are under an angle with the adjacent cuff surface, such that the elastic regions are (also) bending out of plane of the cuff, bending upwards and away from the core (under the cuff).

Due to the elastic profile of the article, the article is typically stored and packed in folded state, typically folded at least twice around transverse folding lines. For example, a preferred diaper herein may be folded twice, around two different transverse lines, to thus obtain a folded diaper of less than ½ of its original unfolded length, e.g. about 1/3 of the original length.

The cuff herein is positioned such that the core is between the cuff and the backsheet, i.e. such that the cuff is positioned adjacent the body facing surface of the backsheet and absorbent core. The cuff is connected to the backsheet, in a manner described above, e.g. typically at least along part of the longitudinal edges of the backsheet and the cuff. The cuff is preferably not directly connected or attached to the core. The cuff is preferably a sheet-like material.

The cuff herein may be liquid pervious or impervious. It may be highly preferred that the cuff is liquid pervious in one direction, but liquid impervious in the opposite direction, e.g. that body fluids may penetrate through the cuff to the remaining part of the diaper, but that no or limited amounts of fluid can penetrate in reverse direction, towards the wearer's skin. For example, the cuff may be treated with a chemical such that it is hydrophilic on one side and hydrophobic at the opposite side, as described hereinafter.

The cuff is preferably fully or partially elasticated, such that it is longitudinally extendible. Preferably, the cuff comprises thereto the elasticated regions, as described above.

Preferred articles herein comprise a cuff which has an elastic profile, based on a two-cycle hysteresis, measured by the method below, using a 500mm/min clamp speed, which is as follows:

1.5Lt by a first load force of less than 1.1N, 3.0Lt by a first load force of less than 2.1N and 4.5Lt by a first load force of less than 3.0N and a second unload force at 4.5Lt of more than 0.9N, a second unload force at 3.0Lt of more than 0.5 and a second unload force at 1.5Lt of more than 0.1N (provided the multitudes of Lt are below 0.8Ltₛ. otherwise the respective force number is irrelevant).

More preferably, the profile of the cuff is:

1.5Lt by a first load force of less than 0.6 N, 3.0Lt by a first load force of less than 1.1N and 4.5Lt by a first load force of less than 1.5N and a second unload force at 4.5Lt of more than 0.9N, a second unload force at 3.0Lt of more than 0.5N and a second unload force at 1.5Lt of more than 0.1N (provided the multitudes of Lt are below 0.8Ltₛ. otherwise the respective force number is irrelevant).

The above elastic profile of the shortened cuff is measured by the following method, measuring the two-cycle hysteresis of said shortened cuff portion (following ASTM 76-96):

The cuff of an article is placed between to clamps in a horizontal tensile tester Z10/LH 1S, as available from Zwick (Ulm, Germany). The clamps are positioned such that exactly the shortened cuff portion is between the clamps and such that exactly and only the shortened cuff length is not covered by the clamps (i.e. the shortened cuff portion being that part of the cuff that belongs to the shortened article (portion), as set out above). The clamps have at least the same size as the width of the cuff in the clamps, so that the clamps at least cover the total width of the cuff in the clamps. The initial clamp distance should then be 4 cm. The measurement is done in a controlled environment, whereby the temperature is kept constant on 23° C (+/- 2° C) and the humidity on 50% (+/- 2%).

The two-cycle hysteresis test is than performed, stretching the shortened cuff (portion) up to 4.5Lt or 0.8Ltₛ, which ever is the lowest value, while measuring the forces applied on the shortened cuff during the stretching at the various stretching stages/ lengths; when 4.5Lt or 0.8Ltₛ is reached, the shortened cuff is kept in that position for 60seconds, before the controlled relaxation back to the original position of the clamps, i.e. 4cm distance (and the unload forces may be measured at the various stages/ lengths); when the original position of clamps is reached, i.e. 4 cm distance, the shortened cuff is held in this position for 60 seconds, before the second cycle starts, stretching the shortened cuff up to 4.5Lt or 0.8Ltₛ, optionally measuring the load forces applied at the various stages/ lengths; when 4.5Lt or 0.8Ltₛ is reached again, the shortened cuff is held in this position for 60 seconds, before the relaxation back to the original position, and the unload forces of this second unload cycle are measured for the various stages/ lengths, as set out in the table above.

In this embodiment of the invention, the value of the first load and second unload forces are believed to b essential to the performance of the cuff and representative for its elastic profile. Measurement of the first unload force and second load force may be performed, but is believed to be less representative for the force profile of the cuff.

The cuff preferably comprises elastic regions with elastic material which have an about similar or the same elastic profile.

The article preferably has an elastic profile, based on a two-cycle hysteresis, measured by the method below, using a 500mm/min clamp speed, which is as follows:

0.25Lₛ by a first load force of less than 0.6 N, 0.55Lₛ by a first load force of less than 5N or even less than 3.5 N and 0.8Lₛ by a first load force of less than 10.0N or even less than 7.ON and a second unload force at 0.55Lₛ of more than 0.4N, and a second unload force at 0.80Lₛ of more than 1.4N or even more than 2.0N.

More preferably, the profile of the article is:

0.25Lₛ by a first load force of less than 0.6 N, 0.40Lₛ by a first load force of less than 1.5N, 0.60Lₛ by a first load force of less than 2.8N, and 0.80Lₛ by a first load force of less than 5.4N and a second unload force at 0.40Lₛ of more than 0.1N, a second unload force at 0.60Lₛ of more than 0.6N and a second unload force at 0.80Lₛ of more than 2.0N.

Even more preferred is that the article has a profile of:

0.25Lₛ by a first load force of less than 0.3N, 0.40Lₛ by a first load force of less than 0.7N, 0.60Lₛ by a first load force of less than 21.4N, and 0.80Lₛ by a first load force of less than 53.2N and a second unload force at 0.40Lₛ of more than 0.3N, a second unload force at 0.60Lₛ of more than 0.7N and a second unload force at 0.80Lₛ of more than 2.0N.

In addition, it may be preferred that the elastic profile of the article is as set out above, but then measured as a two-cycle hysteresis performed with a clamp speed of 10mm/min.

The cuff is preferably compliant, soft feeling, and non-irritating to the wearer's skin. A suitable cuff may be manufactured from a wide range of materials, such as porous foams; reticulated foams; apertured plastic films; or woven or non-woven webs of natural fibers (e.g., wood or cotton fibers), synthetic fibers (e.g., polyester or polypropylene fibers), or a combination of natural and synthetic fibers. If the cuff includes fibers, the fibers may be spunbond, carded, wet-laid, meltblown, hydroentangled, or otherwise processed as is known in the art. One suitable cuff comprising a web of staple length polypropylene fibers is manufactured by Veratec, Inc., a Division of International Paper Company, of Walpole, Massachusetts under the designation P-8. Suitable cuff materials are described in U.S. 3,929,135, U.S. 4,324,246; U.S.4,342,314; U.S. 4,463,045; U.S. 5,006; U.S. 4,609,518 and 4,629,643.

Preferably, the cuff is made of a hydrophobic material or is treated to be hydrophobic in order to isolate the wearer's skin from liquids contained in remaining part of the article. For example, if the cuff is made of a hydrophobic material, preferably at least the upper surface of the cuff is treated to be hydrophilic so that liquids will transfer through the cuff more rapidly. This diminishes the likelihood that body exudates will flow off the cuff rather than being drawn through the opening of the cuff. The cuff can be rendered hydrophilic by treating it with a surfactant or by incorporating a surfactant into the cuff. Suitable methods for treating the cuff with a surfactant include spraying the cuff material with the surfactant and immersing the material into the surfactant. A more detailed discussion of such a treatment and hydrophilicity is contained in US 4,988,344; US 4,988,345.

Any portion of the cuff may be coated with a lotion as is known in the art. Examples of suitable lotions include those described in U.S. 5,607,760; U.S. 5,609,587; U.S. 5,635,191; U.S. 5,643,588; WO 95/24173.

The backsheet is generally that portion of the diaper positioned adjacent the garment facing surface of the cuff and core, which prevents the body fluids or exudates absorbed and contained therein from soiling articles which may contact the diaper, such as bedsheets and undergarments. In preferred embodiments, the backsheet is impervious to liquids (e.g., urine) and comprises a thin plastic film such as a thermoplastic film having a thickness of about 0.012 mm (0.5 mil) to about 0.051 mm (2.0 mils). Suitable backsheet films include those manufactured by Tredegar Industries Inc. of Terre Haute, IN and sold under the trade names X15306, X10962 and X10964. Other suitable backsheet materials may include breathable materials, which permit vapors to escape from the diaper while still preventing exudates from passing through the backsheet.

The backsheet, or any portion thereof, may be elastically extensible in one or more directions, such as described in U.S. 5,518,801.

The backsheet connected to the cuff, and typically the absorbent core, or any other element of the diaper by any attachment means known in the art. The attachment means may include a uniform continuous layer of adhesive, a patterned layer of adhesive, or an array of separate lines, spirals, or spots of adhesive, such as disclosed in U.S.4,573,986. Adhesives that have been found to be satisfactory are manufactured by H. B. Fuller Company of St. Paul, Minnesota and marketed as HL-1620 and HL-1358-XZP. Alternatively, the attachment means may comprise heat bonds, pressure bonds, ultrasonic bonds, dynamic mechanical bonds, or any other suitable attachment means or combinations of these attachment means as are known in the art.

The absorbent core, which is preferably present, may comprise any absorbent material which is generally compressible, conformable, non-irritating to the wearer's skin, and capable of absorbing and retaining liquids such as urine and other certain body exudates, such as comminuted wood pulp, creped cellulose wadding; meltblown polymers, including coform; chemically stiffened, modified or cross-linked cellulosic fibers; tissue, including tissue wraps and tissue laminates; absorbent foams; absorbent sponges; super absorbent polymers; absorbent gelling materials; or any other known absorbent material or combinations of materials. Exemplary absorbent structures for use as the absorbent core are described in U.S. 4,610,678; U.S. 4,673,402; U.S. 4,834,735; U.S. 4,888,231; U.S.5,137,537; U.S. 5,147; U.S. 5,260,345; U.S. 5,387,207; and U.S. 5,625,222.

There may be a cuff or sublayer present, positioned between the core and the cuff, preferably such that at least the part of the core under the opening is covered by this topsheet. This cuff can be made of a the same type of materials as described for the cuff, and it may even be made of the same material as the cuff of the diaper. Suitable materials may also include large cell open foams, macro-porous compression resistant non-woven highlofts, large size particulate forms of open and closed cell foams (macro and/or microporous), highloft non-wovens, polyolefin, polystyrene, polyurethane foams or particles, structures comprising a multiplicity of vertically oriented looped strands of fibers, absorbent core structures described above having punched holes or depressions, and the like. (As used herein, the term "microporous" refers to materials that are capable of transporting fluids by capillary action. The term "macroporous" refers to materials having pores too large to effect capillary transport of fluid, generally having pores greater than about 0.5 mm in diameter and more specifically, having pores greater than about 1.0 mm in diameter.) It may be integral with another element of the diaper or may be one or more separate elements joined directly or indirectly with one or more elements of the diaper. This cuff or sublayer typically serves to grab the fecal matter, and to ensure that the matter traveled though the opening is effectively stored in the remaining part of the article, and not migrate back through the opening, thereby soiling the skin of the wearer.

The article may have side panels, and/ or more preferably elasticized one or more leg cuffs. The diaper preferably further includes leg cuffs, which provide improved containment of liquids and other body exudates. Leg cuffs may also be referred to as leg bands, side flaps, barrier cuffs, or elastic cuffs, as described in; U.S. 3,860,003; U.S. 4,808,178 and 4,909; U.S. 4,695,278 and 4,795,454.

The diapers herein typically has a first or front waist band and a second or back waist band. The waist bands of the diaper herein, when worn, typically gather or encircle the waist of the wearer and are generally at the highest at the highest elevation of the article, when the wearer is in the standing, upright position. The waist region, or preferably the waistband typically comprises the force line of the diaper. In one type of diapers, the waistband is open prior to use and needs fastening around the waist of the wearer. Thereto, the diaper preferably has a fastening system, typically joined to the waist region or band. Preferred fastening systems are described hereinafter in more detail, a most preferred system involving fastening tabs and landing zones, whereof the fastening are part of one waist region and the landing zones are part of the opposite waist region. Diapers herein which serve as pull-on or training pants have typically a waist region, which is a unity, and which is already fastened prior to use. The anal of vaginal cuff is preferably joined to the waistbands.

The diaper may comprise at least one elastic waist feature that helps to provide improved fit and containment. The elastic waist feature is generally positioned in the waistband. It is generally intended to elastically expand and contract to dynamically fit the wearer's waist. The elastic waist feature may be constructed in a number of different configurations including those described in U.S. 4,515,595; U.S. 5, 151,092

Preferred may be that the article, or preferably diaper, includes a topical adhesive or body adhering composition which acts to hold the opening further in place during use. Typically, this is comprised by the cuff or part thereof, so as to further improve the alignment of the opening in the cuff with for example the anus of the wearer. The topical adhesive may be located on the cuff, or the body adhering composition may (also) be integral with the material making up the cuff or other element of the absorbent article or may be a separate material disposed directly or indirectly on all or any portion of the absorbent article. Further, the body adhering composition may be disposed on any portion of the absorbent article in any pattern or configuration including, but not limited to lines, stripes, dots, and the like. In one preferred embodiment, the topical adhesive is present on the elasticated regions along the edges of the opening. Suitable body adhesives are known in the art.

Preferably, the diaper includes a thermally activatable adhesive, which acts to hold the article or some portion thereof in place during use. A "thermally activatable" adhesive is an adhesive that exhibits an increase in "tack" or adhesion after being warmed to a temperature at or above the activation temperature of the adhesive. The "activation temperature" of a thermally activatable adhesive is the temperature at which a the adhesive is activated (i.e., the temperature at which the adhesion of the adhesive increases significantly, as described herein). In certain embodiments wherein the maximum adhesion is achieved over a range of temperatures, the activation temperature is the temperature at which the increase in adhesion begins. Preferably, the activation temperature of the thermally activatable adhesive is between about 28°C and 60°C.

The activatable adhesive may also be thermally deactivatable and/or thermally reversible. A thermally deactivatable adhesive exhibits a decrease in "tack" or adhesion after being cooled to a temperature at or below the deactivation temperature of the adhesive. The "deactivation temperature" of a thermally deactivatable adhesive is the temperature at which the adhesive is deactivated (i.e., the temperature at which the adhesion of the adhesive decreases significantly, as described herein). A thermally reversible adhesive may be activated by an increase in temperature and, subsequently, deactivated by a corresponding decrease in temperature. The "deactivation temperature" of a thermally reversible adhesive is the temperature at which the adhesive is deactivated (i.e., the temperature at which the adhesion of the adhesive decreases significantly, as described herein). The activation temperature and deactivation temperature of thermally reversible adhesives may be the same or different temperatures.

The activation temperature of thermally activated topical adhesives activated by skin temperature will typically be between about 33°C and 38°C, more preferably between about 35°C and 37°C.

The thermally activatable adhesive of the present invention may be a crystallizable polymer or a functional equivalent of a crystallizable polymer having a weight average molecular weight in the range of about 20,000 to 2,300,000 Daltons, typically 100,000 to 1,300,000 Daltons, and more typically 250,000 to 1,000,000 Daltons. Further, the polymer chains in the crystallizable polymer composition may optionally be crosslinked to provide greater physical stability of the adhesive. The adhesive composition may optionally include additives as known in the art, such as filers, tackifiers, antioxidants, and the like. The adhesives of the present invention may be applied to or coated onto any substrate by any means known in the art. Suitable substrates are preferably breathable films as described herein for use as backsheets, polyolefinic films, non-wovens, highlofts, formed films, apertured films, and the like. One exemplary thermally activatable adhesive is described as Example 1 in US Patent 5,387,450. Other examples of thermally activated adhesives suitable for use in the claimed invention are described in more detail in U.S. Patent Nos. 5,156,911 and 5,648,167. An exemplary thermally reversible adhesive is described as Example 2 in the above-referenced US Patent 5,387,450.

It is known in the art to include spacers between on the core, which provide a space between the core and a subsequent layer. In the present invention, it is preferred that no such spacers are provided, e.g. that the cuff can be in contact with the core, but that it can move away from the core, to be no longer in contacts with the core.

### Preferred processes to make the article of the invention

Preferred article of the invention herein are obtained by a process wherein one or more elastic bands or strands is/ are applied parallel to the longitudinal axis of a cuff, along the edges fo the opening therein, e.g. a non-woven material, such that one or more elastic region(s) is/ are obtained which extends along about the whole length L of the article, or even along the whole length of the active cuff, i.e. that part of the cuff which in use acts to receive body exudates, positioned between the force lines of the article, around the waist of the wearer, e.g. the length of the cuff minus the parts which form (part of), or are attached to the waist regions or waistbands. Whilst the elastic material may be applied such that the elastic regions are in the form of two separate substantially parallel elastic zones, or for example in an Y-shaped form or V-shaped form, it is preferred that the elastic material is applied such that an X-shaped elasticated region is obtained. Preferred dimensions of the elastic region are described herein.

The non-woven may already contain an opening along its longitudinal axis, so that then the elastic bands or strands are typically attached to either longitudinal edge of the opening, such that the end portions of the elastic bands or strands bend away from the opposing end portions of the opposing elastic bands or strands, such as to form an X-shaped elastic region. Alternatively, the non-woven may not yet contain an opening, and then, the elastic bands or strands are in a X-shape, prior to attachment to the non-woven, where after a slit opening is cut through both a part of the non-woven and a part of the elastic band/ strands (together referred to as laminate), along the longitudinal axis of the non-woven and the band or strands.

Any method may be used to attach the elasticated region to the cuff, including the methods described above for attaching the backsheet to other parts of the article, and including heat bonding and gluing methods Preferred glues include H2031, available from ATO-Findley and/ or HL-1620 available from H.B. Fuller (St Pauls, USA).

In a non-limiting example, two elastic band of L-89 elastic material, available from Fulflex, with (in relaxed state) a thickness of about 70microns, a width of 20mm and a length of 16cm, are obtained and also a sheet of a polypropylene non-woven, available as P-14 from Veratec, Inc. of Walpole, MA, which is 25 cm wide and 50 cm long (i.e., in the direction of stretch) in a relaxed state.

In relaxed state, a slit opening with a length of 20cm and a width of 4cm is cut in the non-woven.

One elastic band is glued in a stretched state to each longitudinal edge of the opening of the non-woven. This is done such that the middle 11cm (16cm minus areas of 2.5cm at both ends, which are used later for attachment) of the elastic film is stretched to about the length of the non-woven, minus the length of the front and back areas reserved to form the front and back waistband (2x2.5cm) and minus the length needed to attach the elastic film on both sides (2x2.5cm), e.g. 50cm - (2x2.5cm) - (2x2.5cm)= 40cm.

Each elastic band is glued on the non-woven in a manner that the ends of the bands bend away from the ends of the opposing elastic band, in the shape of an X. This is done such that, in after application, the distance between to the ends of the (end portions or regions of the) strands is 80mm in contracted state, whilst the distance between the center point of the strands is only 40 mm, in contracted state.

The angles between the end portions of the strands are as mentioned above, e.g. preferably 40-60°.

Thus, a stretched, elasticated cuff with a slit opening having two elasticated bands along the edges, in the shape of an X is obtained, whereby each end of the elastic film is located 25mm from the edge of the non-woven (i.e., the edge intended to be in the front or back waist region of a finished diaper).

The total length of the cuff is preferably less than 300mm, or even less than 260mm or even les than 240mm; the shortened portion length is preferably less than 160, or even less than 120mm or even less than 100mm.

The cuff may be used on a Pampers Premium Size 4 diaper, designed for a baby weight range of 21-37 pounds, or may replace the cuff of such a diaper. Thereto, the cuff with the elastic bands is attached to the front and back waistbands.

Alternatively, an elastic strand in the shape of an X can be applied to a non-woven cuff, which does not yet comprise a slit opening, in the manner above. Then, after application of the X-shaped elastic band, a slit opening is cut in the elastic/ non-woven laminate, in a manner specified above. Thus, a cuff similar to the cuff above is obtained, which can then be incorporated in a size 4 diaper, as above.

The dimensions and other parameters of the exemplary diaper embodiments described above can be readily modified by one skilled in the art to smaller or larger wearers, including adult wearers.

### Preferred articles as exemplified by the figures

The following description is specifically for diapers, however the invention is equally applicable to absorbent articles such as pull-on or training pants, pant-type diapers, incontinence briefs, incontinence undergarments, absorbent inserts, diaper holders and liners, feminine hygiene garments, however, a preferred embodiment of an absorbent article of the present invention is a pull-on or training pants or diaper.

Preferred articles of the invention are now being described with reference the figures 1, 2, 3 and 4.

Figure 1 is a plan view of the diaper 20 in its stretched state, with portions of the structure being cut away to more clearly show the underlying structure of the diaper 20 and with the portion of the diaper 20 which contacts the wearer facing the viewer. One end portion of the diaper 20 is configured as a first or front waist region 36 of the diaper 20. The opposite end portion is configured as a second or back waist region 38 of the diaper 20. An intermediate portion of the diaper 20 is configured as a crotch region 37, which extends longitudinally between the first and second waist regions 36 and 38. The waist regions 36 and 38 generally comprise those waist portions of the diaper 20 which, when worn, encircle the waist of the wearer. The waist regions 36 and 38 includes elements which can gather about the waist of the wearer to provide improved fit and containment, or which typically can gather around the waist and can be fastened around the waist by use of fastening means, such as tabs 2, which are fastened to landing zones 29. The crotch region 37 is that portion of the diaper 20 which, when the diaper 20 is worn, is generally positioned between the legs of the wearer.

The shortened article portion 70 is the portion of the diaper 20 between the transverse lines 71 and 72, line 71 being the line through the lowest point of the fastening tabs 27 and line 72 being the transverse line through the lowest point of the landing zones. The stretched shortened article length Lₛ in Figure 1 is thus the shortest distance, i.e. parallel to the longitudinal axis x, between the lines 71 and 72 and this equals the length of the backsheet, L_{b}.

The diaper 20 comprises an anal cuff 24, a liquid impervious backsheet 26, and an absorbent core 28 encased between the cuff 24 and the backsheet 26. The cuff may include regions of reduced permeability to fecal material.

The cuff 24 comprises an opening 30, along the longitudinal axis x of the diaper 20.

The anal cuff 24 is configured to receive through the opening 30 fecal exudates and isolate at least a portion, but preferably all fecal matter from the skin of the wearer.

The anal cuff comprises elasticated regions or bands 31, 32 that are positioned along an opening 30 in the cuff 24. The elastic bands 31, 32 have an X-shape, whereby the ends of two opposing elastic regions bend away from one another. The anal cuff 24 and the slit opening 30 are located in alignment with the wearer's anus during use. Preferably, the slit opening 30 in the cuff 24 is located in a target zone of the diaper. The target zone is that portion of the diaper that is configured to directly receive the insult of fecal matter from the wearer and is generally located in the crotch portion of the diaper. Of course if the cuff is a vaginal cuff of a feminine protection article, the cuff is typically located slightly more towards the front waist region.36. In the diapers of the invention, the anal cuff may equally serve to receive urine, so when worn by females, the cuff may in effect be an anal and vaginal cuff at the same time. Particularly, in one non-limiting embodiment, the target zone may extend from about 5 to about 30 centimeters in length along the longitudinal axis x of the diaper with about one fourth of its length extending longitudinally from the lateral axis y of the diaper 20 towards the first or front waist region 36 and the remainder extending longitudinally towards the second or back waist region 38, when measurements are made with the cuff in a fully extended or stretched state. Generally, about 0% to about 40%, preferably 10% to 35% or even 20% to 30% of the slit opening 30 may be located forward of the lateral centerline on the diaper 20.

The slit opening 30 is generally disposed in the target zone along the longitudinal axis x and is defined by two opposing longitudinally extending side edges 40, a front edge 41 and a back edge 42. The front edge 41 is generally located in the crotch region 37 of the diaper 20 towards the first, front region 36, or in the first waist region 36 itself, while the back edge 42 is located in the crotch region 37 near the second waist region 38, or in the second waist region 38 itself. The slit opening 30 includes a length in the longitudinal direction parallel to the longitudinal axis x of the diaper and a width in the lateral direction which is parallel to the lateral axis y of the diaper 20. The length of the slit opening 30 is within the ranges specified above.

The elastic regions 31 and 32 of the anal cuff 21 ensure that the anal cuff 21 and the opening 30 thereof are positioned and remain positioned in the gluteal groove of the buttocks, including the perianal region.

The edge 40 of the slit opening may be held against the wearer ('s skin) allowing the feces to penetrate the slit opening 30 without deflection, via only the elastic forces supplied by the elastic regions 31 and 32, or optionally additionally by use of a body adhering composition, as described above. In any case, it is preferred that the body adhering composition permit vapors to pass (i.e., breathable), be compatible with the skin and otherwise skin friendly. Further, it is preferred that the body adhesive be at least partially hydrophobic, preferably 60%, more preferably 80%, by weight of the adhesive consist of hydrophobic components. However, hydrophilic adhesives are contemplated in certain embodiments of the present invention.

The elasticated regions 31, 32 may be formed by bonding prestretched elastic bands along the longitudinal edges 40 of the slit opening 30, by the method described herein.

The width of the elasticated regions 31, 32 in stretched state, measured laterally from the longitudinal edges 40 of the slit opening 30 is within the ranges specified above. The width of the slit opening in stretched state 30 is preferably also as specified above.

The elastic regions 31, 32 extend from the slit opening 30 in the direction of the waist regions, preferably in a X-shape, with front elastic regions 43 and 44 and/ or back elastic regions 45 and 46.

In stretched state, preferred maximum distance between the elastic regions 32 and 31 is at least 200% of the minimum distance between the elastic regions 31, 32.

The diaper 20 preferably also includes a fastener system, such as a hook-loop fastening system, which typically includes at least one engaging component (male fastening component or fastener) 27, shown in figure 1 to be a fastening tab 27 and at least one landing zone 29 (female fastening component). The diaper 20 may also include such other features as are known in the art including, barrier leg cuffs, gasketing cuffs, front and rear ear panels, waist cap features, elastics and the like to provide better fit, containment and aesthetic characteristics. If present, the barrier leg cuffs are typically at least twice as far removed from the anus or vagina as the edges of the opening of the anal cuff, for example for a 4 size baby, the barrier leg cuff is at least 5cm removed from the anus, while the edge of the opening may be less than 2cm removed from the anus.

The above-mentioned additional features are well known in the art and are described in U.S. Pat. No. 3,860,003; and U.S. Pat. No. 5,151,092.

The opening 30 is located in the cuff 24 such that the fecal exudates pass through the opening into a void space formed between the cuff 24 and the absorbent core 28 and/or other underlying layers such as sub layers, acquisition layers and the like. The void space entraps or encapsulates bodily waste. It is also contemplated that the void space may be formed between two elements of the diaper 20, including but not limited to the cuff 24 and the backsheet 26, an sub layer or acquisition layer and the core 28, the core 28 and the backsheet 26, etc.

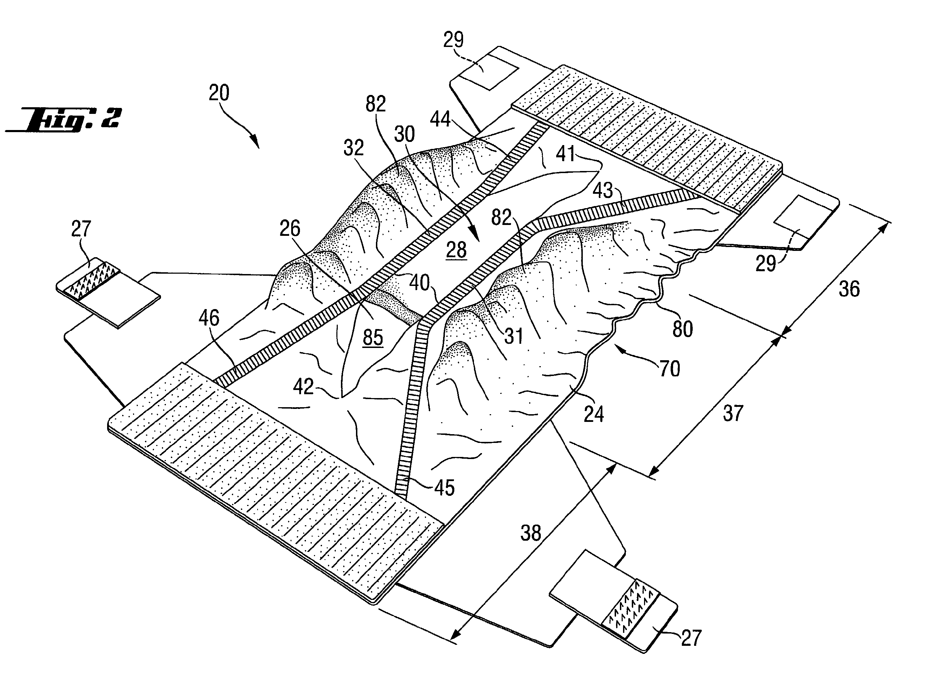

Figure 2 shows the diaper 20 of figure 1 in a perspective view, which shows how the anal cuff 21 is made sag-tolerable and how the cuff, comprising the anal cuff 21, has an extendible width, which is on average about 35% larger than the width of the backsheet 26 and core 28. Thereto, the cuff 24 has longitudinal folds 82, which unfold and thus extend the cuff, when the core 28 or the backsheet 26 of the diaper 20 is pulled down with a small force, e.g. 0.2N, such as the weight of the body exudates received through the anal cuff 21 in the cuff 24 of the diaper 20. Thus, the cuff 24 only extends but does not move away from the wearer's skin, when any downward movement or sagging of the core 28 or backsheet 26 occurs; this is such that the elastic regions 31, 32 keep the anal cuff 21 aligned and positioned against the skin of the wearer, when a force is applied to the diaper.

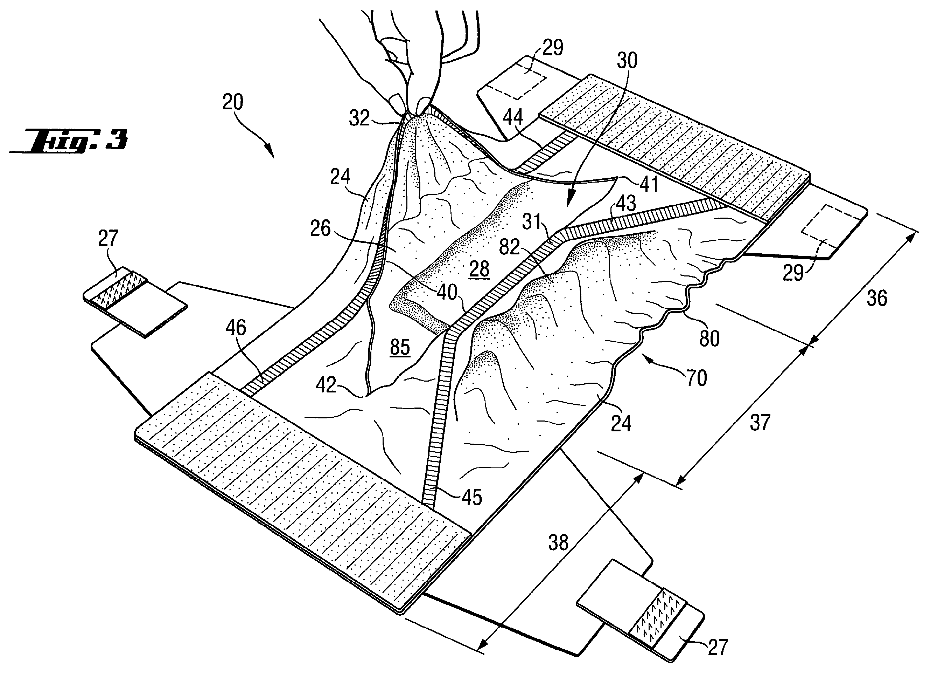

The longitudinal edges of the crotch region of cuff 24 of diaper 20 of figure 2 and 3 are not attached to the core 28 of the diaper 20. They are attached to the backsheet 26 of the diaper 20 and optionally to the leg cuffs, if present and then optionally indirectly connected to the backsheet 26, via the leg cuffs. Preferred may even be that the complete longitudinal edges of the cuff 24 are not attached to the core 28.

Thus, the wider cuff 24 and the limited attachment or no attachment to the core 28 ensures that, when the diaper 20 receives bodily extudates, the elastic regions 30, 31 remain against the skin of the wearer, or in very close proximity to the wearer, even when the core 28 and backsheet 26 are pulled downwards, due to the weight of the exudates received by the diaper 20.

Figure 3 shows in more detail how the cuff 24 of the diaper 20, as shown in figure 1 and 2, can extend by unfolding of the folds 82, e.g. when a small force is applied on the cuff, or typically on the anal cuff 21 therein, and how the anal cuff can remain in position when the backsheet 26 and core 28 sag, e.g. the elasticated regions 31, 32 remain in position.

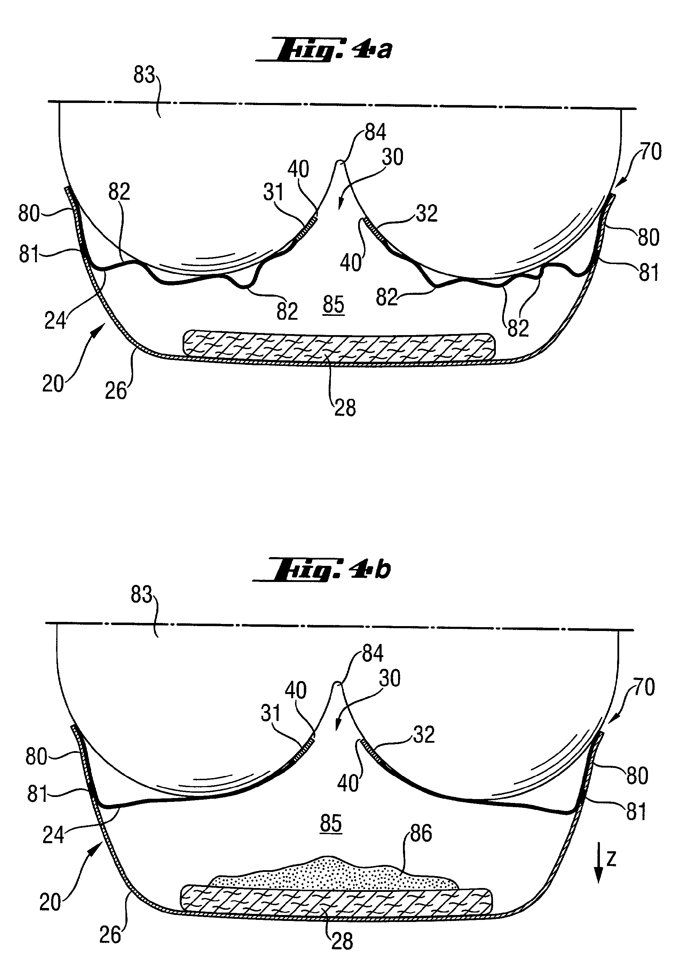

Figures 4a and 4b show how the diaper 20 of the invention performs in use, showing a set of 2 transverse cross sections, along the transverse axis of the diaper.

In figure 4a, the situation is shown that the diaper 20 is applied to the baby 83 and the diaper is not yet soiled. The cuff 24 has elasticated bands 31, 32 which bend away from the void space 85, upwards into or towards the anal groove area 84. Thus, the elastic regions 31, 32 of the anal cuff 24 are positioned around the anal groove 84 and the opening 30 is positioned such as to receive bodily exudates from the anal groove 84.

The cuff 24 contains longitudinal folds 82 when first applied to the baby 83.
The cuff 24 and the backsheet 26 are attached to one another along their longitudinal edges, to form two connecting longitudinal edges or zones 81, one on each side of the diaper 20.
In a preferred embodiment, the diaper 20 comprises also leg cuffs 80, attached to the backsheet and/ or cuff, typically also in the connecting longitudinal edges or zones 81. (For simplicity, only part of the leg cuffs 80 are shown, not including the area where the leg cuffs 80 are in contact with the legs of the baby 83.)

The backsheet 26 is attached to a core 28. The cuff 24 and core 28 are not attached to one another in longitudinal direction.

In figure 4b, the diaper has received bodily exudates 86, through the opening 30 of the anal cuff 21, which has traveled through the void space 85 to the core 28. The core 28 is thus heavier and both the core 28 and the backsheet 26 have moved downwards, in direction z, i.e. have sagged down. Also connecting edges 81 have sagged down. However, the elastic regions 31, 32 and the opening 30 have not moved out of its original position of figure 4a: the cuff 24 has extended in width by defolding the folds 82 and it thereby compensates for the extra distance between the anal groove 84 and the backsheet 26/ core 28, created by the sagging of the core28 / backsheet 26. Moreover, the elastic regions 31, 32 keep the anal cuff 24 aligned and positioned against the skin of the wearer, even when there is a pulling force from the heavier, soiled core 28/ backsheet 26.

## Claims

1. An absorbent article having a backsheet, a core, and a sag-tolerable anal and/ or vaginal cuff, which contains an opening.

2. An absorbent article as in claim 1, which has a shortened article portion, which has a shortened article portion with a stretched shortened article length Lₛ, and whereby the backsheet, cuff and core each have a geometrical center point, whereby the height H₁ from the geometrical center point of the core, which is in a transversely fixed, to the geometrical center point of the cuff, pulled away from the core in the direction of the height H₁ with a force of 1.0N, is at least 0.25Lₛ, preferably at least 0.45Lₛ.

3. An article as in claim 1 which has a shortened article portion, which has a shortened article portion with a stretched shortened article length Lₛ and whereby the backsheet, cuff and core each have a geometrical center point whereby the height H₂ from the geometrical center point of the core, which is in a fixed in position, fixed by its geometrical center point, to the geometrical center point of the cuff, pulled away from the core in the direction of the height H₂ with a force of 1.0N, is at least 0.3Lₛ, preferably at least 0.5Lₛ.

4. An article as in claim 1, which has a shortened article portion, which has a shortened article length L, a stretched shortened article length Lₛ and whereby the backsheet, core and cuff each have a longitudinal axis and a transverse axis and whereby each longitudinal edge of the cuff, or part thereof and a corresponding longitudinal edge of the backsheet is connected to one another to form a longitudinal connection area and whereby the backsheet has a geometrical center point A, and the cuff has a geometrical center point D and whereby each connection area has an inner connection line, being the line closest to said center point A, and an outer connection line, being the closest to the point D, whereby the transverse axis through A intersects the inner connection line in a point B on the backsheet and the transverse axis through D intersects the outer connection line in point C, whereby the distance H₃, being the distance (A to B) + (C to D) is at least 0.3Lₛ, preferably at least 0.5Lₛ.

5. An article as in claim 1 in the form of an infant or adult diaper, which has a shortened article portion, which has a shortened article length L, a stretched shortened article length Lₛ and a contracted shortened article length L_{c}, whereby in use L_{c} is smaller than the shortest distance Lᵣ between the belly button and the small of the back of the infant or adult, preferably L_{c} being at least 20% less than Lᵣ.

6. An absorbent article as in any preceding claim, comprising a backsheet and an anal or vaginal cuff, which is in close proximity to the wearer's skin, having a longitudinal and transverse direction; the cuff being extendible in longitudinal direction and having a means to remain in close proximity to the wearer's skin and; whereby the cuff in relaxed state is also transversely extendible when a force 1N or less is applied to the cuff.

7. An article as in any preceding claim whereby the backsheet and the vaginal or anal cuff each have a longitudinal and transverse direction, whereby the ratio of the longest width of the cuff in transverse direction to Lₛ is at least 1:5, preferably at least 1:4 or even at least 1:3.

8. An article as in any preceding claim whereby the opening of the cuff has longitudinal edges and along said longitudinal edges a means to keep the cuff in close proximity to the wearer's skin, said means preferably being one or more elasticated regions and/ or adhesive regions, and whereby the cuff, in relaxed state, is extendable in transverse and longitudinal direction.

9. An article as claim 8 whereby the article has a contracted shortened article portion with a length L_{c} and whereby the maximum length of the opening is at least 50% of L_{c}, preferably up to 90% of L_{c}, or even from 60% to 80% of L_{c}.

10. An article as in claim 8 or 9 whereby the elasticated regions each have one or more elastic bands longitudinally along said region, having each a width of 5 to 30 mm, preferably 10 to 20mm.

11. An article as in any preceding claim, whereby the backsheet and cuff have each a geometrical center point, the shortest width of the backsheet through its geometrical middle point being Wb and the shortest width of the cuff through its geometrical center point being Wc, and whereby, when the backsheet is attached along its longitudinal axis to a horizontal flat, planar surface and the geometrical center point of the cuff is pulled vertically upwards, with a force along the substantially vertical axis though the geometrical center point of the cuff and backsheet, the force being such that no elastic deformation of the backsheet or cuff occurs, that then the distance H₄ from the geometrical center point of the cuff to the geometrical center of the backsheet is more than Wb-4cm, and/ or that then H₄ is 0.8W_{b} or more.

12. An article as in any preceding claim, whereby the core is positioned between the cuff and backsheet and whereby the core and the cuff are not joined to one another.

13. An article according to any preceding claim whereby the cuff has, in relaxed state, one or more longitudinal folds.

14. An article as in any preceding claim, which is an adult or infant diaper, pull-up pants or training diaper.

15. An article as in any preceding claim, comprising a cuff, positioned between the core and the cuff and positioned at least under the opening of the cuff, preferably positioned over the whole length and width of the core.

16. An absorbent article of claim 1 obtainable by a process comprising the step of:
a) providing a first and second sheet of material, each having a first waist region, second waist region and a crotch region, whereby at least the crotch region of the first sheet is at least 25% wider than crotch region of the second sheet;
b) providing an elastic material;
c) cutting a slit opening in the first sheet of step a), in longitudinal direction, preferably along the longitudinal axis, of the sheet and applying along each longitudinal side of the opening said elastic material over about the total active length of the first sheet, or applying said elastic material over about the total active length of the first sheet in longitudinal direction, preferably along the longitudinal axis, of the sheet, to form a laminate and cutting a slit opening in said laminate;
d) attaching the first and second sheet along at least part of their longitudinal edges, whereby step c) and d) can be in any order.
